# EUROPÄISCHE PATENTSCHRIFT

(11) **EP 1 417 954 B1**
(45) Veröffentlichungstag und Bekanntmachung des Hinweises auf die Patenterteilung: **30.03.2005**
(21) Anmeldenummer: 02090356.3
(22) Anmeldetag: 18.10.2002
(51) Int. Cl.: A61K 7/48

(54) **Ribose-haltige kosmetische Zusammensetzung**
Cosmetic compositions containing ribose
Compositions cosmétiques contenant le ribose

(43) Veröffentlichungstag der Anmeldung: 12.05.2004
(73) Patentinhaber: Heising, Bernhard, 14059 Berlin (DE); Heising, Edelgard, 14059 Berlin (DE); Heising, Cathrin, 14059 Berlin (DE); Heising, Franziska, 14059 Berlin (DE)
(72) Erfinder: Heising, Bernhard, 14059 Berlin (DE); Heising, Edelgard, 14059 Berlin (DE); Heising, Cathrin, 14059 Berlin (DE); Heising, Franziska, 14059 Berlin (DE)

(56) Entgegenhaltungen:
- EP-A- 0 103 878
- EP-A- 0 107 885
- EP-A- 0 818 201
- WO-A-98/46206
- US-A- 3 859 436
- PATENT ABSTRACTS OF JAPAN vol. 1996, no. 11, 29. November 1996 (1996-11-29) & JP 08 175966 A (TEIKA CORP), 9. Juli 1996 (1996-07-09)

## Beschreibung

Hauptanliegen der Hautpflege ist es, die natürliche Funktion der Haut als Barriere gegen schädliche Umwelteinflüsse und gegen den Verlust körpereigener Stoffe zu stärken und wiederherzustellen. Dies ist besonders dann von entscheidender Bedeutung, wenn das natürliche Regenerationsvermögen nicht oder nicht mehr ausreicht.
Die Ribose ( eine Monosaccharid-Pentose) spielt ein zentrale Rolle bei der körpereigenen Synthese von Nucleotiden und anderen metabolischen Produkten.
Die normalerweise bei der reduktiven Biosynthese des sog. Pentosephosphatweges von den Zellen selbst gebildete Ribose wird durch die Umwandlung von Glucose über Pentosephosphat bereitgestellt, wobei die Ribose in Gegenwart einer Ribokinase zu Ribose-5-phosphat phosphoriliert letztlich zur Herstellung von 5-Phospho-ribosyl-1-pyro-phosphat ( PRPP ) führt. Dieses Pyrophosphat ist erforderlich zur Synthese der vorgenannten Nucleotide als Voraussetzung z. B. für die Bereitsstellung des energiereichen Adenosintriphosphates ( ATP ) und z.B. von Adenin und Hypoxanthin sowie von Ribonukleotiden und Desoxyribonucleotiden.
Der nicht-oxidative Pentosephosphatweg ist über die Glyconeogenese reversibel mit der Glycolyse verknüpft, d.h. überschüssige Ribose kann über die Glyconeogenese der Glycolyse wieder zugeführt werden und bei Bedarf auf oxidativem Wege z.B. in ATP umgewandelt werden.
Zucker erfüllen im vorgenannten Zusammenhang also zentrale strukturelle und metabolische Funktionen in den Zellen und Organen. Vorrangig die metabolischen bzw. energetischen Effekte diverser Kohlehydrate, deren Abkömmlinge und/oder Derivate und insbesondere der Ribose, allein oder in Kombination mit anderen Wirkstoffen sind demzufolge umfangreich beschrieben.

Aus der Vielzahl der Veröffentlichungen im Zusammenhang mit der vorrangig energetischen Nutzung von Ribose sollen wirksame Zusammensetzungen aus dem pharmazeutischen-diätetischen Formenkreis nur beispielhaft aufgeführt werden.
Gemäß der DE-OS 17 67 743 wird ausgehend von einer Glucose-haltigen Infusionslösung im Stoffwechsel D-Ribose gebildet, die u.a. am Aufbau der Nucleotide und Nucleinsäuren teilnimmt. Die phosphorylierten Verbindungen ( z.B. Ribose-5-phosphat ) in Verbindung mit gewöhnlichen Zuckerlösungen wirken dabei als metabolische Katalisatoren, sodass der verabreichte Zucker vollständig ausgenutzt wird und nur eine geringe oder überhaupt keine Glucosurie auftritt. Nach WO 200120969 A2 beugt die orale Anwendung von Ribose Muskelkrämpfen und - entzündungen vor. Gemäß DE-OS 196 50 755 A1 führt die Anwendung von D-Ribose sowohl während als auch nach körperlicher Belastung zu einer Verminderung von belastungsassozierten Muskelschmerzen. Eine diätetische Ergänzung zur Steigerung des muskulären Energie-Metabolismus aus Alkanoyl-Carnitin und Ribose oder seinen phophorylierten Analoga ist Gegenstand der WO 200195915 A1.

Die Anwendung von Ribose zur Steigerung der Muskelmasse und zum Abbau von Körperfett mit Dosierungen von ca 10 g pro Tag wird in der WO 200152831 A1 beschrieben, wobei ergänzende Supplemente wie Creatin, L-Carnitin, Pyruvat, Magnesium- und Chromverbindungen zusätzliche Verbesserung bewirken sollen.

Weitere Beispiele für die vorrangig pharmazeutisch orientierte Anwendung der Ribose sind u.a. die Herstellung von Arzeneimitteln zur Behandlung von Leistungsschwächen des menschlichen Körpers wie Organinsuffizienzen, Leistungsschwächen der peripheren Muskulatur sowie der Herzinsuffizienz und nicht-ischämischen Herzerkrankungen sowie zur Schockbehandlung ( DE-OS 42 28 215 A1 ) oder zur Behandlung und/oder Verhütung von Schlaganfällen bzw. Vorformen davon (DE-OS 196 50 754 A1). Die in der WO 200000205 A1 beschriebene Zusammensetzung aus Inositolphosphoglycanen und Ribose soll durch Aktivierung der energieerzeugenden Systeme der Zelle durch "Ankurbeln" des mitochondralen oxidativen Erhaltungssystems zur Vorbeugung oder Behandlung ischämischer Perfusionsdefekte und zur Organkonservierung bei Transplantationen beitragen. Eine vergleichbare Wirkung wird durch eine u.a. Adenosin, Hypoxanthin und Ribose enthaltende kardioplegische Lösung zur Reduzierung der ischämischen Zerstörung des Herzens während der Operation und/oder Transplantation erreicht ( WO 88/02258 A1). Nach US 4.605.644 reduziert die Perfusion von ischämischem Gewebe in Adenin-und Ribose-haltigen verdünnten Salzlösungen die Zeit der Wiederherstellung der Gewebefunktion und des Gewebe-ATP-Wertes.
Triphosphorilierte Zuckerverbindungen, darunter auch die entsprechenden Derivate der D/L-Ribose sollen u.a. in transdermalen Formulierungen zur Verhinderung, Linderung oder Bekämpfung z.B. von Gewebeschäden eingesetzt werden können ( DE 690 33 183 T2 ). Aus der US 4.871.718 A ist bekannt, dass das lokale Auftragen einer Ribose-haltigen Lösung die Wundheilungsrate deutlich erhöht.
Eine u.a. auch zur topischen Anwendung geeignete Zusammensetzung aus einer Ribose-Verbindung (z.B. Ribose, Desoxyribose, andere Ribosederivate und Mischungen davon, mit 35-45 Gew%-Anteil ), beta-Alanin, Ascorbinsäure und Nicotinsäure wird gemäß WO 97/26893 A1 als Mittel zur Krebsbehandlung, vorrangig bei Melanomen eingesetzt. Als weitere, für die metabolische Aktivität der Zellen vorteilhafte Verbindungen werden z.B. ATP, NAD, NADH oder NADP aufgeführt. Ebenfalls topisch, d.h. kosmetisch und dermatologisch angewendet werden Kohlenhydrate oder Kohlenhydrat-Derivate ( u.a. Pentosen als Monosaccharide mit Furanose- oder Pyranose-Ringstrukturen, sowohl als D- und L-Isomere ebenso wie alpha- und beta-Anomere) als antiadhäsive Wirkstoffe zur Prophylaxe und Therapie von durch Mikroorganismen ( DE-OS 195 03 423 A1 ) und Parasiten und Protozoen ( DE-OS 197 21 411 A1 ) verursachte Erkrankungen.

Während wie vorab beschrieben, die pharmazeutisch und diätetisch orientierte Anwendung von Ribose und ihrer metabolischen Abkömmlinge allein oder in Kombination mit anderen Wirkstoffen umfangreich dokumentiert ist, wird der Einsatz von Kohlenhydraten, darunter auch Ribose und ihre Derivate für kosmetische Zwecke nur punktuell beschrieben.
So wird in der DE-OS 15 68 320 von einer in der menschlichen Hornhaut enthaltenen neutralen Zuckerfraktion berichtet, die den wasserretinisierenden Faktor darstellt. Das darauf basierende kosmetische Mittel aus wesentlichen Mengen Fructose und Glucose sowie Mannose, Galactose, Lactose und Psicose soll der erstmalig festgestellten Zusammensetzung des mit Wasser extrahierbaren Hautzuckers entsprechen. In der DE-OS 198 05 827 A1 werden kosmetische oder dermatologische Zubereitungen auf der Basis von Polysacchariden zum Schutze der Haut vor Irritationen, insbesondere zur Verminderung des bei Menschen mit sensibler, empfindlicher oder verletzlicher Haut beobachteten und mit "Stinging" bezeichneten neurosensorischen Phänomens vorgestellt. Hervorgerufen durch stimulierende Umgebungsbedingungen wie z.B. Tensideinwirkung, Wettereinfluß, Wärme- und UV-Strahlung zeigen sich diese störenden neurosensorischen Probleme u.a. in Form von Hautrötung, Kribbeln, Prickeln, Spannen und Brennen der Haut und Juckreiz.

Auf die Behandlung von durch Wasch- und Reinigungsmittel mit stark netzenden und extrahierenden Wirkungen und Chemikalien verursachten Hautproblemen mit einem entsprechenden Hautpflege und -schutzmittel ist das Anliegen der DE-PS 10 25 101 gerichtet. Die physiologischen Schutzfunktionen der Haut sind demnach an in der Hornschicht frei bewegliche und aus ihr mit Wasser extrahierbare Substanzen gebunden, die es gilt durch die erfindungsgemäße Zusammensetzung aus reduzierenden Pentosen wie z.B. Ribose, Desoxyribose, Xylose und Arabinose und einer Aminosäuren wie Glycoll und Histidin entsprechend zu ersetzen.
Die beschriebenen Hautirritationen lassen hier einen engen Zusammenhang mit dem vorgenannten "Stinging"-Phänomen vermuten.
Gegenstand der DE-OS 198 15 090 A1 sind ( Desoxy ) Ribonucleinsäure enthaltende kosmetische Mittel mit deutlich verbesserten hautpflegenden und - schützenden Eigenschaften bei gleichzeitig guter Hautverträglichkeit. Die antioxidative Kapazität der Ribonucleinsäuren soll u.a. die Haut vor entzündlichen Reaktionen schützen. Die Anwendung von Adenosin in kosmetischen und dermatologischen Zubereitungen zur Prophylaxe und Behandlung kosmetischer oder dermatologischer Hautveränderungen ( zB. die Hautalterung ) wird in der DE-OS 195 45 107 A1 beschrieben. Als weitere Mittel gegen das Altern der Haut werden Nucleinsäuren, ihre Bausteine oder Derivate wie z.B. Adenin, Ribonucleotide, Ribonucleoside, Mono-, Di- und Triphosphate von Adenosin, Guanosin, Cytidin etc. eingesetzt ( DE-OS 43 23 615 A1 ). Eine weitere Möglichkeit zur Verhütung und Behandlung der Hautalterung ist schließlich die Anwendung von Oligosacchariden. Dabei handelt es sich z.B. um Lactose, Melobiose und Stachyose oder Gemischen oder Abkömmlingen dieser Zucker mit D-Galactose in nicht reduzierender Endstellung, die als Modulatoren der Synthese und/oder der Ausscheidung der Elastase der Fibroblasten wirken ( DE 694 26 932 T2 ).

PATENT ABSTRACTS OF JAPAN vol. 1996, no 11.29. November 1996 (1996-11-29) & JP 08 175966 A ( TEIKA CORP ) ( 1996-07-09 ). Es werden kosmetische Zubereitungen als Feuchtigkeitsspender beschrieben, die Polysaccharide und somit auch D (-) Ribose enthalten (siehe Zusammenfassung). WO-A-98 46206 (1998-10-22 ) beschreibt kosmetische Zubereitungen in Form von Creme, welche D (-) Ribose enthalten. ( siehe S. 9-16, Beispiel 1-11) und für die Behandlung von Hautirritationen und beschädigter Haut zu verwenden ist (siehe S. 9, 2. Absatz). US-A-3 859 436 (1975-01-22) beschreibt kosmetische Zusammensetzungen enthaltend Ribose (siehe Spalte 3-4, Beispiele 1-8 ). Laut diesem Patent können die kosmetischen Zusammensetzungen als Creme, Lotion, Emulsionen usw. ( siehe Spalte 2- Zeilen 27-31 ) hergestellt werden. EP-A-0 103 878 ( 1984-03-28 ) offenbart ( S.6-8, Beispiele 1-6 ) eine kosmetische Zusammensetzung fur die Behandlung der Kopfhaut. Diese Zusammensetzungen sollen die Regeneration der Kopfhaut verstärken. (S.5): "The topical application of a cosmetic preparation according to the invention (...) has proven to cause a substantial improvement of the local topical conditions and a consequent normalisation of the vitality and reproducing activity of the cells". EP 107 885 ( 1984-03-28 ) offenbart eine dermatologische Zubereitung enthaltend Ribose und Vitamin B1 und B12, welche für die Heilung von Wunden, die Behandlung von Hautentzündungen und Hautausschlägen geeignet ist.

Ausgehend vom vorgenannten Stand der Technik wurde überraschenderweise und nicht vorhersehbar gefunden, dass sich allein mittels einer nicht-phosphorylierten Ribose, und nicht wie oben beschrieben nur in komplexen Kombinationen mit anderen Wirksubstanzen, eine erfindungsgemäße kosmetische Zusammensetzung herstellen läßt, deren Anwendung dazu führt, den Zustand der Haut zu verbessern, die Regenerationsfähigkeit der Zellen zu stärken und deren Stoffwechsel anzuregen. Darüber hinaus wird die Energiebilanz der Hautzellen deutlich verbessert und Prozesse intrazellulärer Schutzmechanismen durch zusätzliche Substratverfügbarkeit unterstützt.
Im Ergebnis umfangreicher Versuche wurde herausgefunden, dass vorrangig die kosmetische Anwendung der D(-)Ribose und ihrer cyclischen Vertreter in Form der alpha- und beta-Pyranose und -Furanose im Konzentrationsbereich von 0,1 bis 20 Gewichts %, vorrangig aber zwischen 0,05 und 5,0 Gewichts % Monosaccharid-Anteil die vorgenannten vorteilhaften energetisch-physiologischen Wirkungen hervorruft.
Unter Einsatz hochreiner Ausgangsprodukte, die mit absoluter Löslichkeit und perfekter Verteilung in Wasser eine wässrige Lösung bilden wurde diese mittels eines Dispensers auf die Hautoberfläche gesprüht, wobei die Hautoberfläche benetzt und die Ribose gleichmäßig verteilt wurde. Die Form der wässrigen Lösung wurde gewählt, um einen eindeutigen Wirksamkeitsbeweis zu erhalten. Der Nachweis des Eindringens der in der Flüssigkeit enthaltenen Ribose in die Hautschichten wurde mit C₁₃-markierter Ribose durchgeführt. Die Messung erfolgte mittels eines Szintilationszählers. Die markierten C-Atome der Ribose waren dabei sowohl zellulär als auch interzellulär eindeutig nachweisbar.
Auf dieser so nachgewiesenen zusätzlichen Substratverfugbarkeit in den Hautzellen beruht letztlich die physiologische Wirksamkeit der Ribose in den erfindungsgemäßen kosmetischen Zusammensetzungen. Zur weiteren Steigerung ihrer Wirksamkeit können diesen Zusammensetzungen zusätzlich bekannte Substanzen wie z.B. Biotin, beigegeben werden.

Die nachfolgenden Beispiele sollen die Anwendungen der erfindungsgemäßen Zusammensetzung näher erläutern, ohne deren Schutzumfang wesentlich einzuschränken.

### Beispiele.

1. Ein 18-jähriger Proband, litt seit seinem 14.Lebensjahr an der schubweisen Entzündung der Gesichtshaut, hat die erfolglose medikamentöse Therapie nach 3 Jahren abgebrochen bzw. ausgesetzt. Mit Beginn der Behandlung wurde das mit 56 kleineren und größeren z.T. entzündeten Pickeln übersäte Gesicht morgens und abends mit einer 0,08 %igen wässrigen Ribose-haltigen Lösung besprüht. Am 2. Tag der Behandlung war die Rötung der entzündeten Stellen besonders stark; das Gesicht war insgesamt leicht gerötet. Zwölf Pickel hatten innerhalb von 12 Stunden eine besonders schnelle Reifung erfahren und waren mit eitrigem Sekret gefüllt. Der Inhalt der Pickel wurde ausgedrückt und abgewischt. Ab dem 4. Tag kam es zum Abklingen der Entzündung. Neun nicht vereiterte entwickelten Pustel, die jedoch nicht größer wurden. Nach 1 Woche waren die Pickel klein und blass. Nach weiteren14 Tagen hatte die Gesichtshaut ihr Aussehen positiv verändert. Die Pickel waren abgeheilt und/oder trocken. Der Proband berichtet, dass in den geheilten Hautregionen eine angenehme, bewegliche Spannung zu bemerken war. In den folgenden 18 Tagen der Behandlung waren auch die letzten Hautirritationen verschwunden.
2. Zwölf weibliche Probanden, die zuvor unterschiedliche Hautpflegeprodukte mit feuchtigkeitsspeichernden Substanzen verwandten, berichteten nach deren Absetzen von Spannungen in der Haut und von einem Trockenheitsgefühl. Nach 14-tägigem Auftragen einer 0,05 %igen wässrigen Ribosezubereitung auf die Gesichtshaut verschwanden diese Irritationen und traten nicht mehr auf. Einige Probanden blieben bei ihrem bisherigen Pflegeverhalten und benutzten zusätzlich das ribosehaltige Kosmetikum. Bei allen Probanden sah die Haut nach 14 Tagen wesentlich frischer,glatter und gut durchblutet aus
3. Bei einem Probanden mit Sonnenbrand hat sich nach dem Auftragen eines 0,05 %igen wässrigen Ribosekosmetikuns die Rötung generalisiert , ohne dass die Haut schmerzte. Bei täglichem Einsprühen verminderte sich die Rötung nach drei Tagen sichtbar, nach 1 Woche verschwanden Rötung und Irritation vollständig, ohne dass es zur sonst üblichen Ablösung der oberen Hautschicht oder zu anderen sichtbaren Schäden kam.
4. Ein Proband mit sog. "Mallorca-Akne" wurde an den betroffenen Hautpartien mit einem 0,08 %igen wässrigen Ribose-Kosmetikums behandelt. Innerhalb von 12 Stunden nach Anwendung verschwinden Rötung und Schwellung vollständig. Irgendswelche Nebenwirkungen wurden nicht beobachtet.
5. Eine Frau hat sich am Herd verbrannt. Das sofortige Aufsprühen einer 0,1 %igen Riboselösung führte schon nach 5 Minuten zum deutlichen Abklingen der Brandschmerzen, ohne dass eine sonst übliche Brandblase entstand. Im Ergebnis der weiteren Behandlung ( 3 bis 4 x tägliches Auftragen ) hatte sich nach 1 Woche ohne Narbenbildung und im nahtlosen Übergang zur umgebenden Haut die neue Haut gebildet.
6. Nach der morgentlichen Rasur wurde die Kinnpartie und das Gesicht von fünf Probanden ohne Anwendung weiterer Pflegemittel mit einem erfindungsgemäßen 0,08 - 0,1 %igen Ribosekosmetikum behandelt. Schon nach 3 Tagen sah die Gesichtshaut deutlich glatter und straffer aus.
7. Auf das Haar und die Kopfhaut von sechs männlichen Probanden mit normaler Fettung der Haut und manifesten Schuppenproblemen wurde über einen Zeitraum von 3 Wochen zweimal täglich eine 0,05 bis 0,08 %igen Ribose-Lösung aufgetragen Schuppen, die durch Irritationen der Kopfhaut entstanden waren, verschwinden, die Haare bekommen Glanz und das Wachstum der Haare wird deutlich stimuliert ( bei intakten Haarfollikeln ).

## Patentansprüche

1. Verwendung von D(-)Ribose und ihren cyclischen Formen zur Herstellung eines Medikamentes gegen Hautirritationen.

2. Verwendung nach Anspruch 1 **dadurch gekennzeichnet, dass** die cyclischen Formen der Ribose alpha- und beta-Pyranose und -Furanose sind.

3. Verwendung nach Ansprüchen 1 und 2 **dadurch gekennzeichnet, dass** die Monosaccharide als wässrige Lösung in Konzentrationen von 0,1 bis 20 Gewichts%, vorzugsweise 0,05 bis 5,0 Gewichts% zubereitet und angewendet werden.

4. Verwendung nach Anspruch 1 bis 3 **dadurch gekennzeichnet, dass** das Medikament als Salbe, Lotion, Spray, Gesichtswasser, Einreibung usw. zubereitet und angewendet wird.

5. Verwendung nach Anspruch 1 bis 4 **dadurch gekennzeichnet, dass** das Medikament gegen Akne zubereitet und angewendet wird.

6. Verwendung nach Anspruch 1 bis 4 **dadurch gekennzeichnet, dass** das Medikament gegen Hautirritation, die durch UVA- und UVB-Strahlung entstehen, zubereitet und angewendet wird.

## Claims

1. Use of D(-) ribose and its cyclical forms for the production of a medical agent as a treatment for skin irritation

2. Use according to claim 1, **characterised by** the fact that the cyclical forms of ribose are alpha and beta pyranose and furanose.

3. Use according to claims 1 and 2, **characterised by** the fact that the monosaccarides are prepared and applied in the form of an aqueous solution in concentrations of 0.1 to 20 weight%, preferably 0.05to 5.0 weight%.

4. Use in accordance with claims 1 to 3, **characterised by** the fact that the medication is prepared and applied in the form of ointments, lotions, spray, facial water, embrocations etc.

5. Use in accordance with claims 1 to 4, **characterised by** the fact that the medication is prepared and applied as a treatment for acne.

6. Use according to claims 1 to 4, **characterised by** the fact that the medication is prepared and applied as a treatment for skin irritation caused by UVA and UVB radiation.

## Revendications

1. Utilisation de D-ribose et de ses formes cycliques pour la fabrication d'un médicament contre les irritations cutanées.

2. Utilisation selon la revendication 1 **caractérisée en ce que** les formes cycliques du ribose sont des pyranoses et des furanoses alpha et bêta.

3. Utilisation selon les revendications 1 et 2 **caractérisée en ce que** les monosaccharides sont préparées et utilisées en tant que solution aqueuse dans des concentrations de 0,1 à 20 et de préférence de 0,05 à 5,0 % en poids.

4. Utilisation selon les revendications 1 à 3 **caractérisée en ce que** le médicament est préparé et utilisé en tant que pommade, lotion, spray, tonique, liniment, etc.

5. Utlisation selon les revendications 1 à 4 **caractérisée en ce que** le médicament et préparé et utlisé contre acné.

6. Utilisation selon les revendications 1 à 4 **caractérisée en ce que** le médicament est préparé et utlisé contre les irritations cutanées causées par les rayons UVA et UVB.
